# EUROPÄISCHE PATENTSCHRIFT

(11) **EP 4 288 783 B1**
(45) Veröffentlichungstag und Bekanntmachung des Hinweises auf die Patenterteilung: **19.03.2025**
(21) Anmeldenummer: 22703624.1
(22) Anmeldetag: 03.02.2022
(51) Int. Cl.: G01N 35/04, G01N 35/00, B01L 3/00, C12M 3/00, B01L 9/06, G01N 35/02

(54) **VERFAHREN ZUM HANDHABEN BIOLOGISCHER ZELLKULTUREN**
METHODS OF HANDLING BIOLOGICAL CELL CULTURES
MÉTHODES DE MANIPULATION DES CULTURES DE CELLULES BIOLOGIQUES

(30) Priorität: 03.02.2021 DE 102021102487
(43) Veröffentlichungstag der Anmeldung: 13.12.2023
(73) Patentinhaber: Aixinno Limited, Cambridge, Cambridgeshire CB24 4FQ (GB)
(72) Erfinder: GILLIGAN, Patrick Clemente, Cambridge Cambridgeshire CB1 3SU (GB); TEO, Kenneth B. K., Cambridge Cambridgeshire CB23 5BH (GB); CONNAH, John Edward, Cambridge Cambridgeshire CB 25 OEJ (GB); BULLINARIA, David Anthony, Bluntisham, Huntingdon PE28 3XD (GB)
(74) Vertreter: Grundmann, Dirk
(86) Internationale Anmeldenummer: PCT/EP2022/052551
(87) Internationale Veröffentlichungsnummer: WO 2022/167510

(56) Entgegenhaltungen:
- EP-A1- 3 142 085
- WO-A1-2020/098958
- JP-A- H02 158 501
- US-A1- 2019 210 818
- US-A1- 2020 333 364

## Beschreibung

### Gebiet der Technik

Die Erfindung betrifft ein Verfahren zum Handhaben von Trägern, die biologische Zellkulturen oder die Hilfsmittel zur Behandlung biologischer Zellkulturen tragen, mit einer einen End Effector, beispielsweise einen Greifer aufweisenden Handhabungseinrichtung, wobei eine Mehrzahl von Trägern jeweils mit ihren Rändern auf Tragelementen aufliegt, die paarweise einander zugeordnet in einem standardisierten vertikalen Abstand von den sich gegenüberliegenden Seitenwänden einer Aufbewahrungseinrichtung abragen, und die Träger aufgrund ihrer Art oder der von ihnen getragenen Hilfsmittel unterschiedliche Aufbewahrungshöhen erfordern, von denen einige größer sind als das Ein- oder Mehrfache des standardisierten vertikalen Abstands, wobei die Träger einen Informationsträger aufweisen, dessen Information von einer der Handhabungseinrichtung zugeordneten Leseeinrichtung auslesbar und von einer Recheneinrichtung auswertbar ist.

### Stand der Technik

Die WO 2020/098958 A1 beschreibt ein System zur Aufbewahrung und zum Handhaben von biologischen Zellkulturen. Die biologischen Zellkulturen werden auf Mikroplatten bevorratet. Die Mikroplatten werden wiederum in Speichermodulen bevorratet. Die Behandlung der Zellkulturen erfolgt mit Hilfsmitteln, beispielsweise mit Flüssigkeiten, Pipettenspitzen und anderen Gegenständen, die auf Trägern bevorratet werden. Die Mikroplatten bilden Träger für die biologischen Zellkulturen. Die Träger für die Hilfsmittel haben denselben Grundriss (Bodenfläche) wie die Träger für die Zellkulturen. Zur Identifikation der Träger sind Informationsträger vorgesehen, die einen Barcode, Text oder RFIDs aufweisen können. Es ist eine Leseeinrichtung vorgesehen, mit der die Information ausgelesen werden kann.

Die Hilfsmittel können verschiedene Aufbewahrungshöhen in Anspruch nehmen. Die Aufbewahrungshöhe hängt im Wesentlichen von der Art des Hilfsmittels ab. Träger, die verschiedene Hilfsmittel tragen, können in einem einheitlich gestalteten Modul mit mehreren, insbesondere regalartigen Aufbewahrungseinrichtungen angeordnet sein. Jede Aufbewahrungseinrichtung besitzt zwei einander zugewandte vertikale Seitenwände, deren Abstand geringfügig größer ist als ein Breiten- oder Längenmaß des Trägers. Von den Seitenwänden ragen Tragelemente ab, auf denen der Rand des Trägers aufgesetzt werden kann. Es sind jeweils paarweise mit einem standardisierten Abstand übereinander angeordnete Tragelemente vorgesehen, auf die jeweils der Rand eines Trägers, eines Rahmens des Trägers oder ein Grundabschnitt des Trägers aufgesetzt werden kann. Die Träger können voneinander verschiedene Aufbewahrungshöhen beanspruchen. Die Aufbewahrungshöhe hängt im Wesentlichen von der Art des Hilfsmittels ab, welches der Träger trägt. Flaschen können beispielsweise eine höhere Aufbewahrungshöhe beanspruchen als flache Schalen. Halterungen für Pipettenspitzen können wiederum eine höhere Aufbewahrungshöhe beanspruchen als Mikroplatten. Träger mit einer hohen Aufbewahrungshöhe beanspruchen eine Vielzahl von jeweils durch den Abstand zweier übereinander angeordneter Tragelemente definierten Fächer. Eine Mikroplatte kann ein einzelnes Fach beanspruchen.

Die US 8,267,310 B2 beschreibt ein Verfahren zur Handhabung eines Speichers für Medikamente.

### Zusammenfassung der Erfindung

Der Erfindung liegt die Aufgabe zugrunde, Maßnahmen anzugeben, mit denen die Fächer der Aufbewahrungseinrichtung optimal genutzt werden können.

Gelöst wird die Aufgabe durch die in den Ansprüchen angegebene Erfindung, wobei die Unteransprüche sowohl vorteilhafte Weiterbildungen der in den nebengeordneten Ansprüchen angegebenen Erfindung als auch eigenständige Lösungen der Aufgabe darstellen.

Zunächst und im Wesentlichen wird vorgeschlagen, dass der Informationsträger eine Angabe über die Aufbewahrungshöhe enthält, die unmittelbar mit der Leseeinrichtung ermittelbar ist. Die Angabe über die Aufbewahrungshöhe kann eine Klartextangabe sein, die über eine OCR-Erkennung auslesbar ist. Die Angabe kann aber auch in einem Barcode kodiert sein. Sie kann auch Informationsgehalt eines RFIDs sein. Wesentlich ist, dass die Angabe ohne Zwischenschaltung externer Tabellen aus sich heraus ermittelbar ist. Die Aufbewahrungshöhe kann die Höhe des Trägers sein. Sie kann aber auch die Gesamthöhe des Trägers inklusive des vom Träger getragenen Hilfsmittels sein. Es ist insbesondere vorgesehen, dass die Handhabungseinrichtung von einer lokalen Steuer- oder Recheneinrichtung gesteuert wird. Diese lokale Steuer- oder Recheneinrichtung ist bevorzugt in der Lage unmittelbar aus der Information des Informationsträgers eine Aufbewahrungshöhe auszulesen. Die Ermittlung der Aufbewahrungshöhe erfolgt somit autark, ohne die Verwendung von externen oder internen Tabellen oder anderweitig gespeicherten Daten, sondern lediglich durch Auslesen der vom Informationsträger getragenen Informationen. Das erfindungsgemäße Verfahren beinhaltet zunächst die Zuordnung von Informationsträgern zu jeweils einem Träger. Der Informationsträger kann verschiedene Informationen tragen, beispielsweise ein Datum, Angaben über den Typ des Trägers, Angaben über den Typ des Hilfsmittels, das vom Träger getragen wird oder Angaben über die Art der biologischen Zellkultur, die vom Träger getragen wird. Die Information kann darüber hinaus einen eindeutigen Identifikationswert tragen oder eine Orientierung angeben. Ferner kann eine Prüfsumme auf dem Informationsträger hinterlegt sein, mit der die Informationen prüfbar sind. Ein Träger kann mehrere Informationsträger tragen, beispielsweise können zwei voneinander verschiedene, insbesondere sich gegenüberliegende Seiten jeweils einen Informationsträger tragen, wobei derartige Informationsträger zusätzlich Informationen über die jeweilige Seite, beispielsweise "Vorderseite" oder "Rückseite" tragen. Wesentlich ist aber, dass der Informationsträger eine Angabe über die Aufbewahrungshöhe enthält. Unter Aufbewahrungshöhe kann eine Höhenangabe verstanden werden, die die Gesamthöhe des Trägers inklusive des von ihm getragenen Hilfsmittels beispielsweise in Zentimetern angibt. Unter Aufbewahrungshöhe kann aber auch eine Angabe über die Anzahl der Fächer der Aufbewahrungseinrichtung sein, die zur Aufbewahrung des Trägers mit dem von ihm getragenen Hilfsmittel verwendet wird. Der Informationsträger kann mit der Information bedruckt werden. Es kann sich um einen Aufkleber handeln, der am Träger befestigt wird. Jeder Träger kann einen individualisierten Informationsträger aufweisen. Der Informationsträger kann auch materialeinheitlicher Bestandteil des Trägers sein, beispielsweise kann der Informationsträger am Oberflächenbereich des Trägers sein, der mit Informationen versehen ist. Beispielsweise können die Informationen auf einen Flächenbereich des Trägers selbst eingeprägt oder eingraviert sein. Der Informationsträger kann neben den Informationen über die Aufbewahrungshöhe auch Informationen über die Art des vom Träger Getragenen enthalten. Der Träger kann einen Grundabschnitt oder einen Rahmen aufweisen. Der Rahmen oder der Grundabschnitt kann Stützschultern ausbilden, mit denen sich der Träger auf den Tragelementen abstützt. Die Tragelemente können Tragleisten sein, die von den beiden sich gegenüberliegenden Seitenwänden geringfügig abragen. Die Tragleisten ragen aber mit einer ausreichenden Weite in den Freiraum zwischen den Seitenwänden, dass der Träger sicher auf den Tragleisten ruhen kann. Zwischen zwei auf derselben Höhe verlaufenden vertikalen Träger, die jeweils einer von zwei aufeinander zu weisenden Seitenwänden einer Aufbewahrungseinrichtung zugeordnet sind, erstreckt sich eine horizontale Freifläche, deren Querschnitt ausreichend groß ist, dass vom Träger Getragenes dort hindurchragen kann. Zwischen vertikal übereinander angeordneten Tragleisten befindet sich ein Abstand von beispielsweise 30 mm, der ein standardisiertes Abstandsmaß darstellt, der das Höhenmaß eines Fachs definiert. Der horizontale Abstand zweier in einer Ebene liegenden Tragleisten ist derart bemessen, dass die Hilfsmittel sich über mehrere Fachhöhen erstrecken können. Der Informationsträger kann an einer definierten Stelle angeordnet sein, beispielsweise kann ein bestimmter Flächenabschnitt eines Grundabschnitts oder eines Rahmens des Trägers den Informationsträger tragen. Es sind bevorzugt ein oder mehrere Module vorgesehen, wobei die Module Einrichtungen sind, in denen zumindest zwei Träger aufgenommen werden können. Eine Handhabungseinrichtung oder ein Greifer oder eine Handhabungseinrichtung mit einem Greifer kann zumindest einem der Module zugeordnet sein. Jedes Modul kann ein oder mehrere Aufbewahrungseinrichtungen aufweisen. Die Aufbewahrungseinrichtung weist bevorzugt eine Entnahmeseite auf. An dieser Entnahmeseite kann der Träger der Aufbewahrungsvorrichtung entnommen werden. Dies erfolgt bevorzugt mit einem End Effector der Handhabungseinrichtung. Der End Effector kann von einer schaufelartigen Platte oder einem Paddel ausgebildet sein, die bzw. das unter den Träger gefahren werden kann, um den Träger zu untergreifen und anzuheben. Es ist aber auch möglich, dass der End Effector ein Greifer mit zangenartig aufeinander zu bewegbaren Fingern ist. Mit den Fingern kann der Träger gegriffen werden. Die Aufbewahrungsvorrichtung kann eine zu einem End Effector gewandte Entnahmeseite aufweisen. Die Aufbewahrungseinrichtungen können um einen Freiraum eines Aufbewahrungsmoduls angeordnet sein. Die Aufbewahrungseinrichtungen können regalartig ausgebildet sein mit mehreren übereinander angeordneten Fächern. Mehrere gleichartige Aufbewahrungseinrichtungen können um den Freiraum angeordnet sein. Die Entnahmeseite weist jeweils zum Freiraum. Innerhalb des Freiraums kann die Handhabungseinrichtung angeordnet sein, die sich in Vertikalrichtung verlagern lässt. Die Handhabungseinrichtung kann einen Greifer aufweisen, der sich in Horizontalrichtung verlagern lässt, um einen Träger aus der Aufbewahrungseinrichtung zu entnehmen oder einen Träger in die Aufbewahrungseinrichtung einzusetzen. Gemäß einer bevorzugten Ausgestaltung der Erfindung weist der Greifer eine Leseeinrichtung auf, mit der die Information des Informationsträgers gelesen werden kann. Beispielsweise kann die Leseeinrichtung ein Barcodescanner sein. Die Leseeinrichtung kann aber auch ein bildabbildendes System, beispielsweise eine Kamera sein, um beispielsweise eine Klartextinformation zu lesen. Die Leseeinrichtung kann aber auch eine Antenne sein, um einen RFID auszulesen. In einer anderen Ausführungsform kann vorgesehen sein, dass der Greifer den Träger zu einer Leseeinrichtung transportiert, welche an anderer Stelle angeordnet ist und welche den Informationsträger auslesen kann. Es kann vorgesehen sein, dass die Leseeinrichtung vom Greifer getrennt ist, wobei unter Greifer jedwede Einrichtung verstanden wird, mit der ein Träger bewegt werden kann.

Gemäß einer Weiterbildung der Erfindung ist vorgesehen, dass ein Näherungssensor vorgesehen ist. Der Näherungssensor kann dem Greifer zugeordnet sein. Er kann der Leseeinrichtung benachbart angeordnet sein. Mit dem Näherungssensor kann erkannt werden, ob ein Fach mit einem Träger, beispielsweise mit einem Rahmen eines Trägers oder einem Grundabschnitt des Trägers belegt ist. Mit dem Näherungssensor kann aber auch erkannt werden, ob ein oberhalb eines von einem Grundabschnitt angeordneten Fachs mit einem Hilfsmittel oder dergleichen belegt ist. Der Näherungssensor kann ein optischer Sensor, ein Ultraschallsensor aber auch ein kapazitiver oder induktiver Näherungssensor sein. Der Näherungssensor kann auch ein Laser-Näherungssensor sein, eine Kamera, insbesondere eine weitere Kamera sein. Es ist aber auch vorgesehen, dass die Leseeinrichtung so ausgebildet, dass sie die Funktion eines Näherungssensors ausüben kann. Der Näherungssensor und die Leseeinrichtung können am Greifer angeordnet sein.

Mit dem erfindungsgemäßen Verfahren lässt sich die Nutzung der Fächer einer Aufbewahrungseinrichtung optimieren. Eine lokale Steuer- oder Recheneinrichtung eines Moduls, das mit mehreren Aufbewahrungseinrichtungen der zuvor beschriebenen Art ausgestattet ist und einen Greifer aufweist, kann den Greifer derart steuern, dass er einzelne Träger aus der Aufbewahrungseinrichtung herausnimmt und an anderer Stelle in dieselbe Aufbewahrungseinrichtung oder einer anderen Aufbewahrungseinrichtung wieder einsetzt. Der Greifer kann mit seiner Leseeinrichtung und mit seinem Näherungssensor zuvor freie Fächer ermitteln. Durch fortgesetztes Herausnehmen einzelner Träger und Wiedereinsetzen der einzelnen Träger an anderer Stelle des Moduls können die unterschiedliche Aufbewahrungshöhen aufweisenden Träger derart angeordnet werden, dass die Gesamtzahl der unmittelbar übereinander angeordneten freien Fächer vergrößert wird. Beispielsweise kann ein einzelnes freies Fach dadurch gefüllt werden, dass mittels des Greifers ein Träger gesucht wird, der eine Aufbewahrungshöhe von einer Höheneinheit aufweist. Dieser Träger kann das einzelne freie Fach auffüllen. Alternativ dazu kann aber auch ein eine Aufbewahrungshöhe von mehreren Höheneinheiten aufweisender Träger, der unmittelbar unterhalb des freien Fachs oder unmittelbar oberhalb des freien Fachs angeordnet ist, herauf- oder herabgesetzt werden, um so das einzelne freie Fach aufzufüllen. In analoger Weise können mehrere übereinander angeordnete freie Fächer dadurch aufgefüllt werden, dass unter Verwendung des Näherungssensors zunächst die Höhe des von den freien Fächern gebildeten Freiraums ermittelt wird. Hierzu fährt der Greifer die Positionen an, an denen üblicherweise ein Informationsträger zu finden ist. Ist kein Informationsträger zu finden und gibt der Näherungssensor kein Signal, so kann davon ausgegangen werden, dass das Fach nicht belegt ist. Auf diese Weise kann eine Anzahl übereinander angeordneter freier Fächer ermittelt werden. Mit der Leseeinrichtung kann dann in den Aufbewahrungseinrichtungen ein Träger gesucht werden, der eine Aufbewahrungshöhe aufweist, die der Höhe der übereinander angeordneten freien Fächer entspricht. Dies erfolgt erfindungsgemäß durch Auslesen des Informationsträgers. Dieser enthält erfindungsgemäß die Angabe über die Aufbewahrungshöhe. Der gefundene Träger kann dann mittels des Greifers in die freien Fächer eingesetzt werden.

Erfindungsgemäß kann auch vorgesehen sein, dass die Aufbewahrungseinrichtungen eines Moduls untereinander gleichgestaltet sind. Die Aufbewahrungseinrichtungen können beispielsweise in der Art eines Karussells angeordnet sein und von einem Drehantrieb gedreht werden. Eine Seitenwand des Moduls, welches ein einheitliches Gehäuse ausbilden kann, kann eine Beladeöffnung aufweisen, mit der das Modul an ein Prozessmodul oder an ein weiteres Speichermodul angrenzt. Durch die Beladeöffnung können Träger von einem Modul in ein benachbartes Modul gebracht werden. Die Beladeöffnung kann in geeigneter Weise verschließbar sein. Jedes von zwei aneinander angrenzenden Modulen kann Beladeöffnungen aufweisen. Die Beladeöffnungen zweier aneinander angrenzender Module fluchten zueinander, so dass Träger durch die beiden geöffneten Beladeöffnungen von einem Modul zu einem anderen Modul gebracht werden können. Die Beladeöffnung kann insbesondere derart verschließbar sein, dass ein Gasaustausch zwischen einer Innenseite des Moduls oder einer Außenseite des Moduls vermindert ist. Die Beladeöffnung kann auch so verschließbar sein, dass ein Wärmefluss zwischen außerhalb des Moduls und innerhalb des Moduls vermindert ist. Das Modul kann beispielsweise ein Inkubator oder ein Gefrierschrank oder ein Kühlschrank sein. Bei einer bevorzugten Ausgestaltung ist vorgesehen, dass eine Anzahl von vier, fünf, sechs, sieben, acht, neun, zehn, elf oder zwölf Aufbewahrungseinrichtungen um einen zentralen Freiraum des Moduls derart angeordnet sind, dass jede Aufbewahrungseinrichtung unmittelbar an eine benachbarte Aufbewahrungseinrichtung angrenzt, so dass ein Transfer eines Trägers von einem Modul zu einem benachbarten Modul durch eine Aufbewahrungseinrichtung erfolgen muss. Um einen Träger von innerhalb des Moduls durch die Beladeöffnung aus dem Modul zu transportieren, muss ein vor der Beladeöffnung liegender Bereich einer Aufbewahrungseinrichtung, beispielsweise eines Speicherregals einen Freiraum ausbilden, durch den der Träger mittels des Greifers hindurch transportiert wird. Dieser Freiraum muss zumindest die vertikale Höhe aufweisen, die der Aufbewahrungshöhe des Trägers entspricht. Mit dem erfindungsgemäß ausgebildeten Greifer kann unter Verwendung der Leseeinrichtung und/oder des Näherungssensors ermittelt werden, ob ein Freiraum mit einer ausreichenden vertikalen Höhe zur Verfügung steht. Für den Fall, dass ein solcher Freiraum nicht zur Verfügung steht wird vorgeschlagen, dass der Greifer so oft Träger aus einem vor der Beladeöffnung liegenden Bereich einer Aufbewahrungseinrichtung entfernt, bis ein Freiraum mit ausreichender vertikaler Höhe zur Verfügung steht, wobei die vertikale Höhe zumindest der Höhe des durch die Beladeöffnung zu transportierenden Trägers entspricht. Es kann vorgesehen sein, dass sich sämtliche Aufbewahrungseinrichtungen eines Moduls grundsätzlich für die Bereitstellung eines Freiraums eignen und die Steuereinrichtung derart eingerichtet ist, dass zur Bildung des Freiraums die Aufbewahrungseinrichtung ausgewählt wird, bei der eine Minimalzahl von Trägern bewegt werden muss, um einen genügend großen Freiraum auszubilden, durch den ein ausgewählter Träger mit der erkannten Aufbewahrungshöhe hindurch bringbar ist.

### Kurze Beschreibung der Zeichnungen

Ausführungsbeispiele der Erfindung werden nachfolgend anhand beigefügter Zeichnungen erläutert. Es zeigen:
- Fig. 1: perspektivisch beispielhaft acht verschiedene Träger zur Verwendung biologischer Zellkulturen,
- Fig. 2: schematisch eine Vorrichtung zur Behandlung biologischer Zellkulturen,
- Fig. 3: den Schnitt durch ein Speichermodul, wie es in der Figur 2 mit den Bezugsziffern 12, 13 und 14 dargestellt ist,
- Fig. 4: schematisch ein Speicherregal 19 und eine Handhabungseinrichtung 22 mit einem Greifer (End Effector) 23, der eine Leseeinrichtung 24 und einen Näherungssensor 25 aufweist,
- Fig. 5: schematisch einen Bereich zweier Module 12, 14 im Bereich einer die beiden Module 12, 14 miteinander verbindenden Beladeöffnung 26,
- Fig. 6: vergrößert einen Ausschnitt aus zwei nebeneinander angeordneten Aufbewahrungseinrichtungen 19.

### Beschreibung der Ausführungsformen

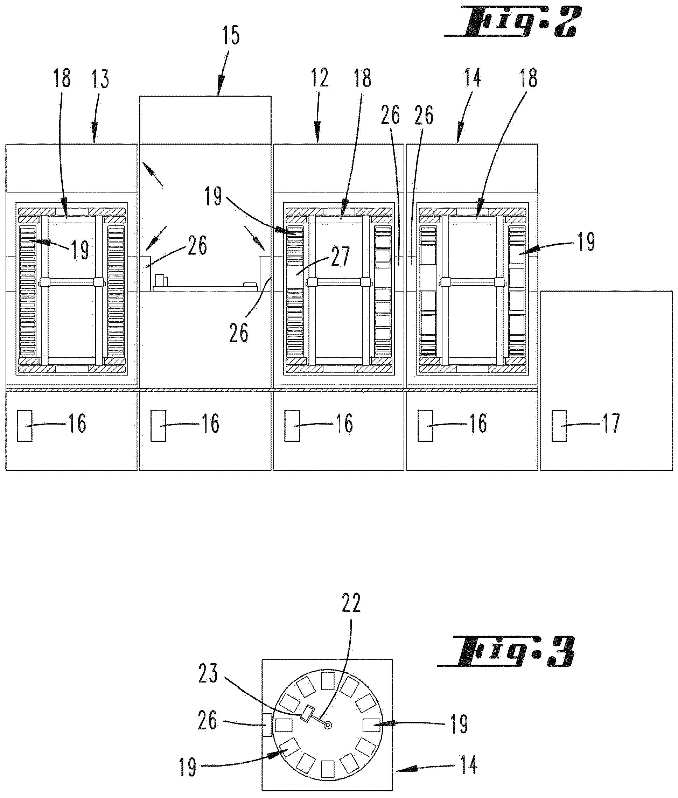

Die Figur 2 zeigt schematisch in der Art einer Ansicht drei Speichermodule 12, 13, 14 zur Aufnahme von standardisierten Trägern, die Zellkulturen oder Hilfsmittel zu deren Behandlung aufnehmen können. Ein Speichermodul 12 dient zur Aufnahme von Hilfsmitteln zur Behandlung von Zellkulturen. In diesem Speichermodul 12 können aber grundsätzlich auch Mikroplatten, die Zellkulturen tragen, aufgenommen werden. Zwischen dem Speichermodul 12 und dem Speichermodul 13, welches ein Inkubator ist, zur Aufnahme von Zellkulturen, befindet sich ein Prozessmodul 15. In diesem Prozessmodul 15 können die Zellkulturen mittels Pipetten oder dergleichen behandelt werden. Das Speichermodul 14 ist ein Kühlschrank, in dem Behandlungsflüssigkeiten bevorratet werden können, mit denen die Zellkulturen behandelt werden können. Die Module 12-14 sowie das Prozessmodul 15 besitzen jeweils eine lokale Steuereinrichtung, die von einer Steuer- oder Recheneinrichtung 16 ausgebildet ist. Die lokalen Steuereinrichtungen 16 können mit einer zentralen Steuereinrichtung 17 in einer Datenaustauschverbindung stehen.

Die Speichermodule 12, 13, 14 sind untereinander gleichgestaltet und unterscheiden sich im Wesentlichen nur durch das in ihnen erzeugte Klima. Im Inkubator 13 kann eine hohe Temperatur herrschen. Im Kühlschrank 14 kann eine niedrige Temperatur herrschen. Die Speichermodule 12, 13, 14 sind untereinander mit Beladeöffnungen 26 verbunden. Die Speichermodule besitzen jeweils zumindest eine oder zwei Beladeöffnungen 26. Die Beladeöffnung 26 eines Speichermoduls 12 kann unmittelbar an die Beladeöffnung 26 eines benachbarten Speichermoduls 14 angrenzen, so dass bei geöffneten Beladeöffnungen 26 Träger 1-9 von einem Speichermodul 12 in das andere, benachbarten Speichermodul 14 transportiert werden. Es ist insbesondere vorgesehen, dass Beladeöffnungen 26 unmittelbar aneinander angrenzender Speichermodule unmittelbar miteinander verbunden sind. Es ist auch vorgesehen, dass Beladeöffnungen 26 eines Speichermoduls 12 unmittelbar an ein Prozessmodul angrenzen. Die Beladeöffnungen 26 können verschlossen werden.

Die Figur 3 zeigt den Querschnitt durch eines der Speichermodule 12, 13, 14. In der Art eines Karussells sind mehrere Aufbewahrungseinrichtungen 19 um ein Zentrum angeordnet. Die Aufbewahrungseinrichtungen 19 können um das Zentrum verdreht werden, so dass die Aufbewahrungseinrichtung 19 in eine Fluchtlage zu einer Beladeöffnung 26 bringbar ist.

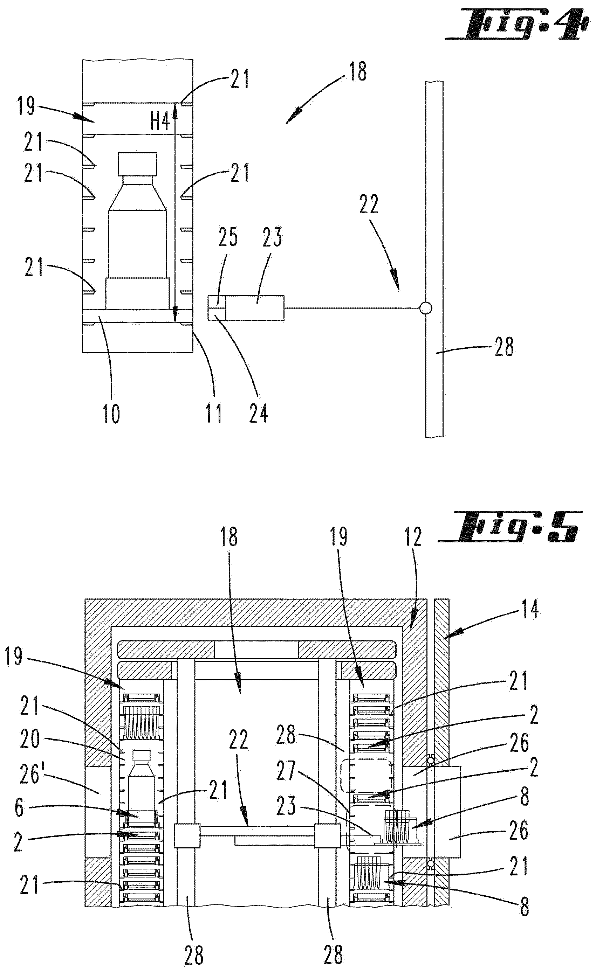

Die Figur 4 zeigt grob schematisch den Aufbau einer Aufbewahrungseinrichtung 19. Die Aufbewahrungseinrichtung 19 bildet ein Speicherregal, in dem, in mehreren Etagen übereinander angeordnet, Träger 1-9 abgelegt werden können. Die Aufbewahrungseinrichtung 19 besitzt zwei Seitenwände, die parallel zueinander verlaufen. Auf den aufeinander zu weisenden Seitenwänden sind Tragelemente 21 insbesondere in Form von Tragleisten angebracht. Der horizontale Abstand der Tragleisten 21 kann 5 cm bis 15 cm oder aber auch 20 cm bis 35 cm betragen. Der horizontale Abstand der Tragleisten 21 ist bevorzugt an die Länge bzw. an die Breite einer Standardmikroplatte (85,48 mm x 127,76 mm) angepasst. Der vertikale Abstand der Tragleisten 21, die sich paarweise gegenüberliegen, kann geringfügig größer sein als die Höhe des Trägers 1, 2, der die geringste vertikale Höhe aufweist.

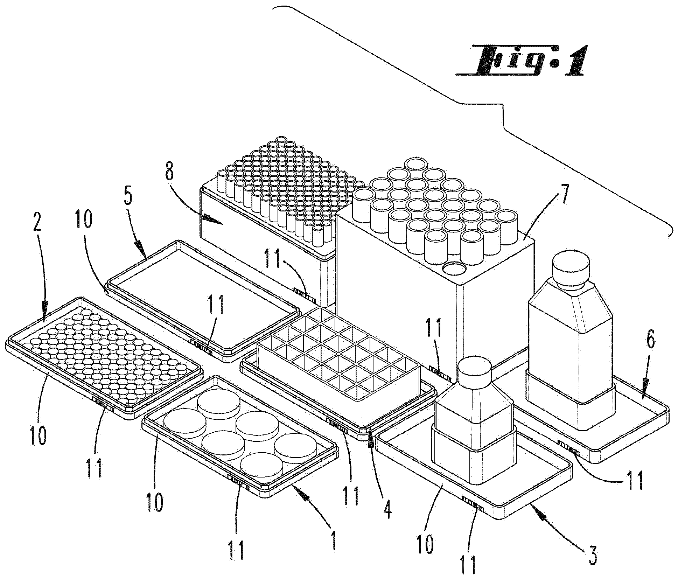

Die Figur 1 zeigt beispielhaft verschiedene Träger 1-9, die zur Aufnahme von Zellkulturen oder Hilfsmitteln zu deren Behandlung dienen. Die Träger 1, 2 sind flache, rechteckige Körper mit Ausnehmungen, in denen biologische Zellkulturen angeordnet sein können. Derartige Träger können eine Höhe von 21 mm aufweisen. Der Träger 1 besitzt einen Grundabschnitt 10, bei dem es sich um einen Grundkörper handeln kann, der eine flache Gestalt aufweist, deren Höhe geringer ist als der Abstand zweier Tragleisten 21. Dieser Träger 3 trägt eine Flasche, mit einer Behandlungsflüssigkeit, die beispielsweise im Kühlschrank 14 untergebracht werden kann.

Der Träger 4 trägt eine Vielzahl von Behältnissen zur Aufnahme von Flüssigkeiten oder Gerätschaften oder dergleichen. Die vertikale Höhe des Trägers 4 ist größer als der vertikale Abstand zweier unmittelbar übereinander angeordneter Tragleisten 21.

Der Träger 5 ist eine flache Schale und besitzt eine Höhe, die geringer ist als der vertikale Abstand zweier unmittelbar benachbarter Tragleisten 21. Der Träger 5 ist insbesondere ein einzelner Container, in welchem biologische Zellkulturen wachsen können oder in welchem eine Flüssigkeit bevorratet werden kann.

Der Träger 6 ähnelt dem Träger 3. Er trägt eine Flasche, die ein größeres Volumen aufnehmen kann, als die vom Träger 3 getragene Flasche.

Der Träger 7 trägt eine Vielzahl von Pipettenspitzen oder länglichen Behältern zur Aufnahme von Flüssigkeiten. Die Höhe des Trägers 7 ist größer als der vertikale Abstand zweier unmittelbar benachbarter Tragleisten.

Der Träger 8 trägt eine Vielzahl von Pipettenspitzen oder anderweitigen länglichen Behältern zur Aufnahme von Flüssigkeiten. Die Höhe des Trägers 8 ist größer als der vertikale Abstand zweier unmittelbar benachbarter Tragleisten 21.

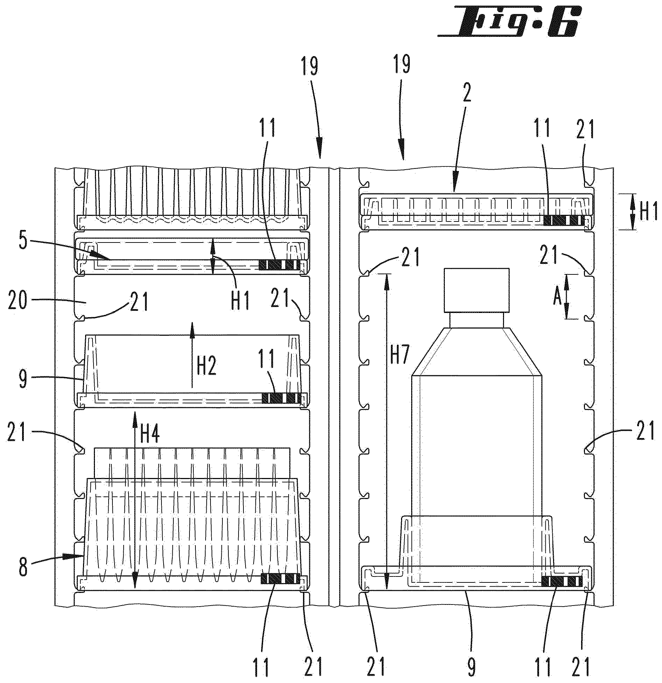

Die Figur 6 zeigt zusätzlich einen Träger 9, der ähnlich dem Träger 5 gestaltet ist, jedoch eine eine zentrale Ausnehmung umgebenden Rand aufweist, dessen Höhe größer ist als der vertikale Abstand zweier benachbarter Tragleisten. Er kann eine Höhe von 30 mm bis 40 mm haben.

Die vorstehenden Ausführungen dienen der Erläuterung der von der Anmeldung insgesamt erfassten Erfindungen, die den Stand der Technik zumindest durch die folgenden Merkmalskombinationen jeweils auch eigenständig weiterbilden, wobei zwei, mehrere oder alle dieser Merkmalskombinationen auch kombiniert sein können, nämlich:

Alle Träger 1-9 weisen einen unteren Abschnitt 10 auf, der ein Grundabschnitt ist und der an einer standardisierten Stelle einen Informationsträger 11 trägt. Der Informationsträger 11 ist an der Außenwand des Trägers 1-9 angeordnet. Der Informationsträger 11 ist derart angeordnet, dass er von einer zentralen Höhlung, die von mehreren Aufbewahrungseinrichtungen 19 umgeben ist, sichtbar ist. Es kann vorgesehen sein, dass jeder Träger 1-9 einen einzigen Informationsträger 11 aufweist. Es kann aber auch vorgesehen sein, dass jeder oder einzelne Träger 1-9 mehrere Informationsträger 11 aufweisen.

Bei weiteren Ausführungsbeispielen der Erfindung kann vorgesehen sein, dass voneinander wegweisende Seiten des Trägers 1-9 jeweils einen Informationsträger 11 aufweisen. In anderen Ausführungsbeispielen der Erfindung kann vorgesehen sein, dass jede Seite eines Trägers 1-9 einen Informationsträger 11 aufweist. Bei Ausführungsbeispielen, bei denen die Träger 1-9 mehrere Informationsträger 11 an voneinander verschiedenen Seiten aufweisen, kann vorgesehen sein, dass jeder Informationsträger 11 auch eine Information über die jeweilige Seite des Trägers 1-9 aufweist, also Informationen darüber, ob es sich beispielsweise um eine kurze Seite, eine lange Seite und/oder eine Front- oder Rückerseite des Trägers handelt. Bevorzugt ist es lediglich erforderlich, zwischen Front- und Rückseite unterscheiden zu können. Der Informationsträger kann ein Flächenabschnitt einer Kunststoff-Oberfläche eines den Träger bildenden Kunststoffkörpers sein.

Die Figur 6 zeigt einige Träger 2, 5, 6, 8, 9, die voneinander verschiedene Höhen aufweisen. Lediglich die Träger 2, 5 weisen eine Aufbewahrungshöhe H₁ auf, die geringer ist als der vertikale Abstand zweier Tragleisten 21.

Der Abstand zweier unmittelbar übereinanderliegender Tragleisten 21 definiert einen Standardabstand A. Der Träger 9 weist eine Aufbewahrungshöhe H₂ auf, die geringer ist als das Doppelte eines Standardabstands A. Der Träger 8 besitzt eine Aufbewahrungshöhe H₄, die größer ist als das Dreifache eines Standardabstandes, aber geringer ist als das Vierfache eines Standardabstandes A, so dass der Träger 8 eine Aufbewahrungshöhe H₄ aufweist, die vier Fächer 20 beansprucht, wobei jedes Fach 20 durch den Raum definiert ist, den zwei übereinanderliegende Tragleisten 21 umgeben. Der Träger 6, der eine Flasche trägt, hat eine Aufbewahrungshöhe H₇ und beansprucht sieben übereinanderliegende Fächer 20.

Der in der Figur 4 schematisch dargestellte Greifer 23 ist in der Lage, beispielsweise mit einer einen Träger 1-9 untergreifenden Platte oder mit Greifarmen einen Träger 1-9 zu greifen, um ihn aus der Aufbewahrungseinrichtung 19 herauszuziehen und ihn entweder in eine andere Position in der Aufbewahrungseinrichtung 19 zu bringen oder den Träger 1-9 durch die Beladeöffnung 26 in eine benachbarte Aufbewahrungseinrichtung 19 eines anderen Moduls 12-14 zu bringen.

Der Greifer 23 oder die Handhabungseinrichtung 22 können eine Leseeinrichtung 24 aufweisen. Mit dieser Leseeinrichtung 24 kann die Information des Informationsträgers 11 gelesen werden. Gemäß einem bevorzugten Ausführungsbeispiel ist die Information als Barcode auf dem Informationsträger 21 aufgebracht. Die Leseeinrichtung 24 kann ein Barcodeleser sein, mit dem die Information des Barcodes gelesen werden kann. Der Barcode kann in einem Abstand innerhalb 10 mm, 20 mm oder 30 mm von der Unterkante des Grundabschnitts 10 des Trägers 1-9 angeordnet sein. Er ist lesbar, wenn sich der Träger 1-9 in der Aufbewahrungseinrichtung befindet. Der Grundabschnitt 10 liegt dabei direkt auf den Tragleisten 21 auf. Da sämtliche Träger 1-9 denselben Grundriss, nämlich bevorzugt den einer Mikroplatte aufweisen, können in alle Aufbewahrungseinrichtungen 19 Träger 1-9 mit unterschiedlicher Aufbewahrungshöhe angeordnet werden.

In nicht dargestellten Ausführungsbeispielen kann die Leseeinrichtung 24 an einer anderen Stelle innerhalb des Speichermoduls 14 angeordnet sein. Um mit einer derartigen, nicht am Greifer 23 oder der Handhabungseinrichtung 22 angeordneten Leseeinrichtung 24 den Informationsträger 11 lösen zu können, wird der den Informationsträger 11 tragende Träger 1-9 zur Leseeinrichtung 24 transportiert.

Die Information des Informationsträgers 11 enthält eine Angabe unmittelbar über die Aufbewahrungshöhe H₁ bis H₇. Es kann sich um eine absolute Höhenangabe handeln, die in Zentimetern angegeben ist. Es kann sich aber auch um eine Höhenangabe handeln, die lediglich ein Vielfaches eines Standardabstands A oder Höheneinheiten angibt. Wesentlich ist, dass die Höhenangabe unmittelbar aus der Information des Informationsträgers 11 ohne Zuhilfenahme weiterer Tabellen oder dergleichen entnehmbar ist. Als Folge dessen kann eine lokale Steuer- oder Recheneinrichtung 16, die jedem der Module 12-14 zugeordnet ist, aus der von der Leseeinrichtung 24 gewonnenen Information eine Angabe über die Höhe eines Trägers 1-9 erhalten. Die Informationen, die der Informationsträger 11 trägt können darüber hinaus ein Datum sein, der Typ des Hilfsmittels (Labware) sein. Die zusätzlichen Informationen können ferner eine Seriennummer, ein einheitlicher Identifikator, eine Orientierung oder eine Checksumme sein. Aus der Typenkennzeichnung kann die Recheneinrichtung 16 beispielsweise eine Flaschengröße oder die Größe einer Pipettenspitze ermitteln.

Der Barcode des Informationsträgers 11 kann das Format LLLLLLL CCCCCCC UUUUUU O H C haben. Wobei dann LLLLLLL eine Chargennummer ist, CCCCCCC eine Katalognummer, UUUUUU eine Seriennummer, O eine Orientierung, H die Höhe oder die Anzahl von in Anspruch genommenen Standardabständen und C eine Checksumme ist. Es sind auch andere Ausgestaltungen möglich. In einer Alternative kann der Barcode das Format DDDDDDFFFFUUUUUUOHHCC aufweisen, wobei DDDDDD ein Tagesdatum, beispielsweise ein Fertigungsdatum ist. Das Tagesdatum kann das Format YYYYWW aufweisen, wobei YYYY das Jahr und WW die Kalenderwoche beispielsweise der Herstellung ist. FFFF kann der Typ des Hilfsmittels sein. UUUUUU kann die Seriennummer sein. O kann die Orientierung sein. HH kann die Höhe sein und CC kann eine Checksumme sein.

Der Greifer 23 der Handhabungseinrichtung 22 kann nicht nur eine Leseeinrichtung 24, sondern auch einen Näherungssensor 25 tragen. Während mit der Leseeinrichtung die Information des Informationsträgers 11 also insbesondere der Strichcode des Barcodes gelesen werden kann, kann mit dem Näherungssensor 25 festgestellt werden, ob ein Fach zwischen zwei übereinander angeordneten Tragleisten 21 belegt ist.

Mit der Leseeinrichtung 14 in Kombination mit dem Näherungssensor 25 kann eine Kontrollfunktion ausgeübt werden. Ermittelt der Näherungssensor 25 einen in einem Fach angeordneten Grundabschnitt 10, und ermittelt die Leseeinrichtung 24 dort einen Barcode, so erkennt die lokale Steuereinrichtung 16, dass es sich hier um den unteren Abschnitt eines Trägers handelt. Aus der in der Information des Informationsträgers 11 enthaltenen Höhenangabe erkennt die Steuereinrichtung 16 wie viele Fächer oberhalb des Grundabschnitts 10 von demselben Träger belegt sind. Mit dem Näherungssensor 25 kann überprüft werden, ob dies der Fall ist und ob die Information des Informationsträgers hinsichtlich der Aufbewahrungshöhe H₁ bis H₇ stimmt. Darüber hinaus kann mit der Kombination einer Leseeinrichtung 24 und einem Näherungssensor 25 festgestellt werden, ob ein Fach frei ist. Es ist auch möglich einen fehlerhaften Informationsträger 11 zu erkennen, wenn ein an sich freies Fach oberhalb der Aufbewahrungshöhe des Trägers nicht frei ist und keinen Barcode trägt.

Mit der erfindungsgemäßen Kennzeichnung der Träger 1-9 mit einem insbesondere als Barcode ausgebildeten Informationsträger 11, der eine explizite Angabe über eine Aufbewahrungshöhe H₁ bis H₇ aufweist, kann jedes Speichermodul 12-14 autark arbeiten. Es ist keine externe Datenverbindung erforderlich, um festzustellen, welche Aufbewahrungshöhe ein Träger 1-9 besitzt. Es ist somit möglich, festzustellen, ob für einen Transfer eines Trägers 1-9 durch die Beladeöffnungen 26', 26 zwischen zwei benachbarten Modulen 12-14 eine Transferöffnung 27 in einer der Aufbewahrungseinrichtungen 19 zur Verfügung steht, durch die der Träger 1-9 hindurch transportiert werden kann. Die Figur 5 zeigt beispielsweise rechts eine Beladeöffnung 26, die eine größere vertikale Erstreckung aufweist als die sich dahinter befindliche Transferöffnung 27 zwischen einem Träger 2 und einem Träger 8. Die vertikale Höhe der Transferöffnung 27 ist hier ausreichend groß, um eine Pipettenspitzenbox 8 hindurch zu transportieren. Die Höhe der Transferöffnung 27 reicht jedoch nicht aus, um beispielsweise einen Träger 9 mit einer hohen Flasche zu transportieren. Um letzteres zu ermöglichen ist es zuvor erforderlich, die wirksame Höhe der Transferöffnung 27 dadurch zu vergrößern, dass der Träger 2 aus dem Bereich der Beladeöffnung 26 entfernt wird. Hierzu wird der Träger 2 vom Greifer 23 gegriffen und an eine andere, geeignete Position innerhalb des Moduls gebracht.

In der in der Figur 5 links dargestellten Situation müssen unterhalb des Trägers 9 angeordnete flache Träger 2 zunächst entfernt werden, damit der Träger 9 mit der Flasche durch die Beladeöffnung 26' hindurch transportiert werden kann.

Die Erfindung betrifft somit ein Verfahren, das dadurch gekennzeichnet ist, dass mithilfe der Leseeinrichtung 24 und/oder oder auch ohne des Näherungssensors 25 die vertikale Höhe einer Transferöffnung 27 ermittelt wird und die vertikale Höhe der Transferöffnung 27 gegebenenfalls durch Entfernen von dort angeordneten Trägern 1-9 und Anordnen der Träger 1-9 in anderen Aufbewahrungseinrichtungen 19 oder an anderen Positionen derselben Aufbewahrungseinrichtung 19 vergrößert wird. Bei diesem Verfahren wird zunächst einer der im Speichermodul 12 angeordneten Träger 1-9 identifiziert, der aus dem Speichermodul 2 entnommen werden soll. Mit der Leseeinrichtung 24 wird sodann die Aufbewahrungshöhe H₁ bis H₇ des Trägers 1-9 ermittelt. Mit der so ermittelten Aufbewahrungshöhe H₁ bis H₇ wird die erforderliche vertikale Höhe der Transferöffnung 27 berechnet. Mit der Leseeinrichtung 24 und gegebenenfalls dem Näherungssensor 25 wird sodann die tatsächliche vertikale Höhe der Transferöffnung 27 ermittelt. Ist die tatsächliche vertikale Höhe der Transferöffnung 27 geringer als die Aufbewahrungshöhe H₁ bis H₇ des ausgewählten Trägers 1-9, so wird durch Entnehmen von im Bereich der Beladeöffnung 26 angeordneten Trägern 1-9 die vertikale Höhe der Transferöffnung 27 vergrößert.

Mit der erfindungsgemäßen Kennzeichnung der Träger 1-9 mit einem insbesondere als Barcode ausgebildeten Informationsträger 11, der eine explizite Angabe über eine Aufbewahrungshöhe H₁ bis H₇ aufweist, kann sich ein Speichermodule 12-14 auch selbsttätig organisieren. Dies kann sogar ohne die Verwendung des Näherungssensors 25 erfolgen. Mit der Leseeinrichtung 24, die bevorzugt am Greifer 23 befestigt ist, aber auch an einem gesonderten Träger, der unabhängig vom Greifer 23 bewegt werden kann, können die Positionen der Grundabschnitte 10 aller in einem Speichermodul 12 angeordneten Träger 1-9 ermittelt werden. Ein Speichermodul 12 kann eine Vielzahl von Aufbewahrungseinrichtungen 19 aufweisen, in denen sich Träger 1-9 befinden. Mittels der Informationen über die Aufbewahrungshöhe H₁ bis H₇ kann die lokale Recheneinrichtung 16 die tatsächlich belegten Fächer 20 ermitteln und insbesondere berechnen, an welcher Stelle sich freie Fächer 20 befinden. Durch eine Entnahme der Träger 1-9 und eine Anordnung an einer anderen Stelle im selben Speichermodul 12 lässt sich die Anzahl freier Einzelfächer 20 vermindern. Durch Umsetzen der Träger 1-9 an andere Positionen kann die Anzahl der mehrere freien Fächer umfassenden Freiräume zur Anordnung von Trägern 1-9 mit großer Aufbewahrungshöhe H₁ bis H₇ vergrößert werden.

Die Erfindung betrifft somit ein Verfahren, das dadurch gekennzeichnet ist, dass mithilfe der Leseeinrichtung 24 die Anzahl freier Einzelfächer 20 vermindert wird oder die Anzahl von unmittelbar aneinander angrenzenden freien Einzelfächer 20 vergrößert wird, in dem Träger 1-9 an andere Positionen gesetzt werden.

Die Erfindung betrifft darüber hinaus ein Verfahren und eine entsprechend ausgebildete Vorrichtung, dass nur mit Hilfe einer Leseeinrichtung und eines Greifers sowie der Steuer- und Recheneinrichtung 16 die in einem Speichermodul angeordneten Träger 1-9 organisiert werden können. Mit der Leseeinrichtung kann das unterste Fach ermittelt werden, das von einem Träger ausgefüllt wird. Durch die Ermittlung der Aufbewahrungshöhe lässt sich dann das nächste über dem Träger liegende nicht von einem von dem Träger getragenen Gut beanspruchte Fach ermitteln. Mit der Leseeinrichtung kann dann festgestellt werden, ob dieses Fach frei ist und ob weitere darüberliegende Fächer frei sind. Mit dem Greifer kann sodann der unter freien Fächern liegende Träger gegriffen werden und derart nach oben versetzt werden, dass unmittelbar über dem Träger keine freien Fächer vorhanden sind. Beginnt man ein derartiges Verfahren mit dem zuoberst liegenden Träger, so werden nacheinander sämtliche freien Fächer ausgefüllt. Die Erfindung betrifft somit auch ein Verfahren, bei dem nacheinander entweder oben oder unten beginnend zwischen zwei unmittelbar übereinander angeordneten Trägern freie Fächer ermittelt werden und diese durch Umverlagerung des darunterliegenden oder darüberliegenden Trägers gefüllt werden.

Die Erfindung betrifft ferner ein Verfahren, mit dem unter Verwendung der Leseeinrichtung 24 und der auf den Informationsträger 11 angeordneten Informationen über die Aufbewahrungshöhe ein Speicherplatz gefunden wird, der eine vorgegebene Mindestanzahl von unmittelbar übereinander angeordneten Einzelfächern 20 aufweist.

In der vorstehenden Beschreibung werden unter dem Begriff Greifer 23 oder Handhabungseinrichtung jede Art von Mittel verstanden, die es alleine oder in Kombination mit anderen Mitteln ermöglichen, einen Träger von einer ersten Position in eine andere, zweite Position zu bewegen. Unter dem Begriff Greifer oder Handhabungseinrichtung wird somit insbesondere auch ein End Effector verstanden. Unter dem Begriff Greifer oder Handhabungseinrichtung kann darüber hinaus auch eine elektrisch betätigbare und von einer Steuereinrichtung steuerbare Vorrichtung verstanden werden, mit der ein Träger 1-9 aus einem Fach 20 entnommen werden kann bzw. in ein Fach 20 hineingesetzt werden kann. Unter Greifer 23 bzw. Handhabungseinrichtung kann aber auch eine Vorrichtung verstanden werden, mit der ein Träger 1-9 aus einem Fach 20 genommen werden kann, indem die Vorrichtung oder ein Teil der Vorrichtung eine Horizontalbewegung durchführt. Die Vorrichtung kann auch eine Vertikalbewegung durchführen, um den Träger 1-9 anzuheben bzw. abzusenken. Der Greifer 23 bzw. die Handhabungseinrichtung kann darüber hinaus von einer Vorrichtung ausgebildet sein, mit der ein Träger 1-9 durch zwei aneinander angrenzende Beladeöffnungen 26 hindurch von einem Speichermodul 12 in ein Speichermodul 14 gebracht werden kann oder ein Träger 1-9 zwischen einem Speichermodul 12, 14 und einem Prozessmodul 15 transportiert werden kann.

Die Erfindung umfasst auch solche Ausführungsbeispiele, bei denen eine Mehrzahl von Speichermodulen 12-14 zumindest eine Handhabungseinrichtung 22 aufweist, wobei die Handhabungseinrichtung 22 in der Lage ist, jeweils zumindest einen Träger 1-9 zu greifen bzw. zu transportieren. Die Erfindung betrifft somit auch ein Verfahren mit ein oder mehreren Modulen 12-14, von denen zumindest eines eine Handhabungseinrichtung 22 aufweist.

## Patentansprüche

1. Verfahren zum Handhaben von Trägern (1-9), die biologische Zellkulturen oder die Hilfsmittel zur Behandlung biologischer Zellkulturen tragen, mit ein oder mehreren Modulen (12-14) von denen zumindest eines eine Handhabungseinrichtung (22) aufweist, wobei eine Mehrzahl von Trägern (1-9) jeweils mit ihren Rändern auf Tragelementen (21) aufliegt, die paarweise einander zugeordnet in einem standardisierten vertikalen Abstand (A) von den sich gegenüberliegenden Seitenwänden einer Aufbewahrungseinrichtung in horizontaler Richtung abragen, und die Träger (1-9) aufgrund ihrer Art oder der von ihnen getragenen Hilfsmittel unterschiedliche Aufbewahrungshöhen (H₁-H₇) erfordern, von denen einige größer sind als das Ein- oder Mehrfache des standardisierten vertikalen Abstands (A), wobei die Träger (1-9) einen Informationsträger (11) aufweisen, dessen Information von einer Leseeinrichtung (24) auslesbar und von einer Recheneinrichtung (16) auswertbar ist, **dadurch gekennzeichnet, dass** die Informationen Angaben über die Aufbewahrungshöhe (H₁-H₇) des jeweiligen Trägers (1-9) enthalten und die Handhabung der Träger (1-9) unter Verwendung dieser Angaben erfolgt.

2. Verfahren nach Anspruch 1, **dadurch gekennzeichnet, dass** der Informationsträger (11) an einem zwei auf die Tragelemente (21) auflegbare Stützschultern aufweisenden Grundabschnitt (10) oder Rahmen des Trägers (1-9) derart angeordnet sind, dass die Information von einer Entnahmeseite der Aufbewahrungseinrichtung (19) auslesbar sind.

3. Verfahren nach einem der vorhergehenden Ansprüche, **dadurch gekennzeichnet, dass** die Handhabungseinrichtung (22) einen Näherungssensor (25) aufweist, mit dem ermittelt wird, ob ein vertikal oberhalb eines Paares von Tragelementen (21) und bis zum unmittelbar darüberliegenden Paar von Tragelementen (21) sich erstreckendes Fach (20) belegt ist und/oder ob auf einem Paar von Tragelementen (21) ein Träger (1-9) aufliegt.

4. Verfahren nach Anspruch 3, **dadurch gekennzeichnet, dass** unter Verwendung der Leseeinrichtung (24) und des Näherungssensors (25) von Trägern (1-9) belegte Fächer (20) und freie Fächer (20) erkannt werden und für das Hinzufügen eines Trägers (1-9) in eine Aufbewahrungseinrichtung (19) eine zumindest der Aufbewahrungshöhe (H₁-H₇) des Trägers (1-9) entsprechende vertikale Anordnung freier Fächer (20) gefunden wird.

5. Verfahren nach Anspruch 4, **dadurch gekennzeichnet, dass** die vertikale Anordnung freier Fächer (20) ausschließlich durch Verwendung der Leseeinrichtung (24) und des Näherungssensors (25) gefunden wird.

6. Verfahren nach einem der vorhergehenden Ansprüche, **dadurch gekennzeichnet, dass** die in einer Aufbewahrungseinrichtung (19) angeordneten Träger (1-9) derart den ihnen zugeordneten Fächern (20) entnommen und anderen Fächern (20) zugeordnet werden, dass sich neue Anordnungen von Fächern (20) bilden, bei denen die Anzahl unmittelbar übereinander angeordneter freier Fächer (20) vergrößert wird.

7. Verfahren nach einem der vorhergehenden Ansprüche, wobei die Aufbewahrungseinrichtung (19) in einem Gehäuse (12-14) zwischen einer Beladeöffnung (26) des Gehäuses (12-14) und der Handhabungseinrichtung (22) angeordnet ist, **dadurch gekennzeichnet, dass** ein vertikaler Bereich (27) der Aufbewahrungseinrichtung (19) auf Höhe der Beladeöffnung (26) freigehalten wird, dessen Höhe der größten Aufbewahrungshöhe (H₁-H₇) eines in dem Gehäuse (12-14) angeordneten Trägers (1-9) entspricht.

8. Verfahren nach einem der vorhergehenden Ansprüche, **dadurch gekennzeichnet, dass** die Leseeinrichtung (24) der Handhabungseinrichtung (22) zugeordnet ist oder in zumindest einem der Module (12-14) angeordnet ist, wobei die Module (12-14) eingerichtet sind, um eine Vielzahl von Trägern (1-9) zu bevorraten.

9. Vorrichtung zur Durchführung eines Verfahrens gemäß einem der vorhergehenden Ansprüche mit zumindest einer Aufbewahrungseinrichtung (19) mit paarweise in einem standardisierten vertikalen Abstand (A) von Seitenwänden abragenden Tragelementen (21), mit einer Mehrzahl von biologischen Zellkulturen oder Hilfsmitteln zur Behandlung biologischer Zellkulturen tragenden Trägern (1-9) und mit einer Handhabungseinrichtung (22) zum Handhaben der Träger (1-9), wobei die Träger (1-9) jeweils einen Informationsträger (11) aufweisen, dessen Information von einer Leseeinrichtung (24) auslesbar und von einer Recheneinrichtung (16) der Einrichtung auswertbar ist, **dadurch gekennzeichnet, dass** die Informationen Angaben über die Aufbewahrungshöhe (H₁-H₇) des jeweiligen Trägers (1-9) enthalten und eine insbesondere programmierbare Steuereinrichtung (16) vorgesehen ist, die so eingerichtet ist, dass die Handhabung der Träger (1-9) unter Verwendung dieser Angaben erfolgt.

10. Vorrichtung nach Anspruch 9, **dadurch gekennzeichnet, dass** der Handhabungseinrichtung (22) ein Greifer (23) zugeordnet ist und/oder dass die Leseeinrichtung (24) der Handhabungseinrichtung (22) oder dem Greifer (23) zugeordnet ist.

11. Träger zur Durchführung eines Verfahrens gemäß einem der Ansprüche 1 bis 8 oder zur Verwendung in einer Vorrichtung gemäß Anspruch 9 oder 10, mit einem Grundabschnitt (10) zur Auflage auf die Tragelemente (21) und einem Informationsträger (11), **dadurch gekennzeichnet, dass** die Informationen des Informationsträgers (11) explizite Angaben über die Aufbewahrungshöhe (H₁-H₇) des jeweiligen Trägers enthalten.

12. Träger nach Anspruch 11, **dadurch gekennzeichnet, dass** die Informationen ein Datum, Angaben über den Typ des Trägers (1-9), einen eindeutigen Identifikationswert, eine Orientierung und/oder eine Prüfsumme und/oder ein "Batch-Number" und/oder einen Wert für eine Höhe enthalten.

13. Vorrichtung nach Anspruch 9 oder 10, Verfahren nach einem der Ansprüche 1 bis 8 oder Träger nach Anspruch 11, **dadurch gekennzeichnet, dass** der Informationsträger (11) einen Barcode trägt und die Leseeinrichtung ein Barcodescanner ist.

14. Vorrichtung nach Anspruch 9, 10 oder 13, Verfahren nach einem der Ansprüche 1 bis 8 oder 13 oder Träger nach Anspruch 11 oder 13, **dadurch gekennzeichnet, dass** ein Träger (1-9) mehrere Informationsträger (11) aufweist und/oder dass zwei an einander angrenzende oder sich gegenüberliegende Seiten jeweils einen Informationsträger (11) aufweisen, und/oder dass die Informationsträger (11) von Trägern (1-9), die mehrere Informationsträger (11) aufweisen zusätzlich eine Information über die Seite, an der der jeweilige Informationsträger (11) befestigt ist, aufweisen.

## Claims

1. A method for handling supports (1-9), which carry biological cell cultures or auxiliary agents for treating biological cell cultures, with one or several modules (12-14), of which at least one has a handling device (22), wherein a plurality of supports (1-9) each rests with its edges on support elements (21), which are allocated to each other in pairs and protrude at a standardized vertical distance (A) from the opposing side walls of a storage device in a horizontal direction, and the supports (1-9), depending on their type or the auxiliary agents they support, require different storage heights (H₁-H₇), some of which are greater than one or more times the standardized vertical distance A, wherein the supports (1-9) have an information carrier (11), the information of which can be read by a reading device (24), and can be evaluated by a computing device (16), **characterized in that** the information contains details about the storage height (H₁-H₇) of the respective support (1-9), and these details are used for handling the supports (1-9).

2. The method according to claim 1, **characterized in that** the information carrier (11) is arranged on a base section (10) or frame of the support (1-9) having two support shoulders that can be placed on the support elements (21), such that the information can be read from a removal side of the storage device (19).

3. The method according to one of the preceding claims, **characterized in that** the handling device (22) has a proximity sensor (25), which is used to determine whether a compartment (20) extending vertically above a pair of support elements (21) and up to the pair of support elements (21) lying thereover is occupied, and/or whether a support (1-9) is resting on a pair of support elements (21).

4. The method according to claim 3, **characterized in that** the reading device (24) and the proximity sensor (25) are used to recognize compartments (20) occupied by supports (1-9) and free compartments (20), and to find a vertical arrangement of free compartments (20) corresponding to at least the storage height (H₁-H₇) of the support (1-9) so as to add a support (1-9) in a storage device (19).

5. The method according to claim 4, **characterized in that** the vertical arrangement of free compartments (20) is found exclusively by using the reading device (24) and the proximity sensor (25).

6. The method according to one of the preceding claims, **characterized in that** the supports (1-9) arranged in a storage device (19) are removed from the compartments (20) to which they are allocated and allocated to other compartments (20) in such a way as to form new arrangements of compartments (20), in which the number of free compartments (20) arranged one directly above the other is increased.

7. The method according to one of the preceding claims, wherein the storage device (19) is arranged in a housing (12-14) between a loading opening (26) of the housing (12-14) and the handling device (22), **characterized in that** a vertical area (27) of the storage device (19) is kept free at the height of the loading opening (26), the height of which corresponds to the largest storage height (H₁-H₇) of a support (1-9) arranged in the housing (12-14).

8. The method according to one of the preceding claims, **characterized in that** the reading device (24) is allocated to the handling device (22), or is arranged in at least one of the modules (12-14), wherein the modules (12-14) are set up to stock a plurality of supports (1-9).

9. The method for implementing a method according to one of the preceding claims, with at least one storage device (19) with support elements (21) that protrude in pairs at a standardized vertical distance (A) from side walls, with a plurality of biological cell cultures or auxiliary agents for treating supports (1-9) carrying biological cell cultures, and with a handling device (22) for handling the supports (1-9), wherein the supports (1-9) each have an information carrier (11), the information of which can be read by a reading device (24), and can be evaluated by a computing device (16) of the device, **characterized in that** the information contains details about the storage height (H₁-H₇) of the respective support (1-9), an in particular programmable control device (16) is provided, which is set up in such a way that the handling of supports (1-9) takes place using these details.

10. The device according to claim 9, **characterized in that** a gripper (23) is allocated to the handling device (22), and/or that the reading device (24) is allocated to the handling device (22) or gripper (23).

11. A support for implementing the method according to one of claims 1 to 8 or for use in a device according to claim 9 or 10, with a base section (10) for placement on the support elements (21) and an information carrier (11), **characterized in that** the information of the information carrier (11) contains explicit details about the storage height (H₁-H₇) of the respective support.

12. The support according to claim 11, **characterized in that** the information contains a date, details about the type of support (1-9), a unique identification value, an orientation and/or a checksum and/or a "batch number" and/or a value for a height.

13. The device according to claim 9 or 10, the method according to one of claims 1 to 8, or the support according to claim 11, **characterized in that** the information carrier (11) carries a barcode, and the reading device is a barcode scanner.

14. The device according to 9, 10 or 13, the method according to one of claims 1 to 8 or 13, or the support according to claim 11 or 13, **characterized in that** a support (1-9) has several information carriers (11), and/or that two adjacent or opposing sides each have an information carrier (11), and/or that the information carriers (11) of supports (1-9) having several information carriers (11) additionally have information about the side to which the respective information carrier (11) is fastened.

## Revendications

1. Méthode de manipulation de supports (1-9) qui portent des cultures de cellules biologiques ou des moyens pour traiter des cultures de cellules biologiques, comprenant un ou plusieurs module(s) (12-14) desquels au moins un présente un dispositif de manipulation (22), dans lequel une pluralité de supports (1-9) repose respectivement par ses bords sur des éléments porteurs (21) qui, agencés par paires entre eux dans un écart (A) vertical normalisé, font saillie dans le sens horizontal à partir des parois latérales opposées d'un dispositif de conservation, et les supports (1-9), en raison de leur type ou des moyens qu'ils portent, exigent différentes hauteurs de conservation (H₁-H₇), desquelles certaines sont plus grandes qu'une fois ou plus l'écart (A) vertical normalisé, dans lequel les supports (1-9) présentent un support d'information (11) dont l'information peut être lue par un dispositif de lecture (24) et évaluée par un dispositif de calcul (16), **caractérisé en ce que** les informations contiennent des indications sur la hauteur de conservation (H₁-H₇) du support (1-9) respectif et la manipulation des supports (1-9) a lieu en utilisant ces indications.

2. Procédé selon la revendication 1, **caractérisé en ce que** le support d'information (11) est disposé sur une section de base (10) présentant deux épaulements d'appui pouvant être posés sur les éléments porteurs (21) ou un cadre du support (1-9), de sorte que l'information puisse être lue d'un côté de prélèvement du dispositif de conservation (19).

3. Procédé selon l'une quelconque des revendications précédentes, **caractérisé en ce que** le dispositif de manipulation (22) présente un capteur d'approche (25) avec lequel il est déterminé si un compartiment (20) s'étendant verticalement au-dessus d'une paire d'éléments porteurs (21) et jusqu'à la paire d'éléments porteurs (21) se trouvant directement dessous est occupé et/ou si un support (1-9) repose sur une paire d'éléments porteurs (21).

4. Procédé selon la revendication 3, **caractérisé en ce que** des compartiments occupés (20) par des supports (1-9) et des compartiments libres (20) sont détectés en utilisant le dispositif de lecture (24) et le capteur de proximité (25), et pour ajouter un support (1-9) dans un dispositif de conservation (19), un agencement vertical de compartiments libres (20) correspondant au moins à la hauteur de conservation (H₁-H₇) du support (1-9) est trouvé.

5. Procédé selon la revendication 4, **caractérisé en ce que** l'agencement vertical de compartiments libres (20) est trouvé exclusivement en utilisant le dispositif de lecture (24) et le capteur de proximité (25).

6. Procédé selon l'une des revendications précédentes, **caractérisé en ce que** les supports (1-9) disposés dans un dispositif de conservation (19) sont ainsi prélevés des compartiments (20) qui leur sont attribués et attribués à d'autres compartiments (20) que de nouveaux agencements de compartiments (20) se forment, dans lesquels le nombre de compartiments (20) libres disposés directement l'un sur l'autre est agrandi.

7. Procédé selon l'une des revendications précédentes, dans lequel le dispositif de conservation (19) est disposé dans un logement (12-14) entre une ouverture de chargement (26) du logement (12-14) et le dispositif de manipulation (22), **caractérisé en ce qu'**une zone verticale (27) du dispositif de conservation (19) à hauteur de l'ouverture de chargement (26) est maintenue libre, dont la hauteur correspond à la plus grande hauteur de conservation (H₁-H₇) d'un support (1-9) disposé dans le logement (12-14).

8. Procédé selon l'une des revendications précédentes, **caractérisé en ce que** le dispositif de lecture (24) est attribué au dispositif de manipulation (22) ou disposé dans au moins un des modules (12-14), dans lequel les modules (12-14) sont conçus pour conserver une pluralité de supports (1-9).

9. Dispositif pour exécuter un procédé selon l'une des revendications précédentes comprenant au moins un dispositif de conservation (19) comprenant des éléments porteurs (21) agencés par paires dans un sens horizontal d'un écart (A) vertical normalisé et faisant saillie de parois latérales, comprenant une pluralité de supports (1-9) portant des cultures de cellules biologiques ou des moyens pour traiter des cellules de cultures biologiques et comprenant un dispositif de manipulation (22) pour manipuler les supports (1-9), dans lequel les supports (1-9) présentent respectivement un support d'information (11) dont l'information peut être lue par un dispositif de lecture (24) et évaluée par un dispositif de calcul (16) du dispositif, **caractérisé en ce que** les informations contiennent des indications sur la hauteur de conservation (H₁-H₇) du support (1-9) respectif, et un dispositif de commande (16) pouvant en particulier être programmé est prévu, qui est conçu de telle sorte que la manipulation des supports (1-9) a lieu en utilisant ces indications.

10. Dispositif selon la revendication 9, **caractérisé en ce que** le dispositif de manipulation (22) a un grappin (23) attribué et/ou que le dispositif de lecture (24) est attribué au dispositif de manipulation (22) ou au grappin (23).

11. Support pour exécuter un procédé selon l'une des revendications 1 à 8 ou pour l'utilisation dans un dispositif selon la revendication 9 ou 10, comprenant une section de base (10) pour l'appui sur les éléments porteurs (21) et un support d'information (11), **caractérisé en ce que** les informations du support d'information (11) contiennent des indications explicites sur la hauteur de conservation (H₁-H₇) du support respectif.

12. Support selon la revendication 11, **caractérisé en ce que** les informations contiennent une date, des indications sur le type du support (1-9), une valeur d'identification unique, une orientation et/ou une somme de vérification et/ou un numéro de lot et/ou une valeur d'une hauteur.

13. Dispositif selon la revendication 9 ou 10, procédé selon l'une des revendications 1 à 8 ou support selon la revendication 11, **caractérisé en ce que** le support d'information (11) porte un code-barres et le dispositif de lecture est un scanner de code-barres.

14. Dispositif selon la revendication 9, 10 ou 13, procédé selon l'une des revendications 1 à 8 ou 13 ou support selon la revendication 11 ou 13, **caractérisé en ce qu'**un support (1-9) présente plusieurs supports d'information (11) et/ou que deux côtés adjacents l'un à l'autre ou opposés présentent respectivement un support d'information (11), et/ou que les supports d'information (11) de supports (1-9) qui présentent plusieurs supports d'information (11), présentent en plus une information sur le côté sur lequel le support d'information (11) respectif est fixé.
